# EUROPEAN PATENT APPLICATION

(11) **EP 3 777 930 A1**
(43) Date of publication of application: **17.02.2021**
(21) Application number: 19774760.3
(22) Date of filing: 27.03.2019
(51) Int. Cl.: A61M 5/28, A61M 5/315

(54) **PRE-FILLED SYRINGE**

(30) Priority: 29.03.2018 JP 2018066114
(71) Applicant: TERUMO Kabushiki Kaisha, Tokyo 151-0072 (JP)
(72) Inventor: OKUDA, Yuji, Fujinomiya-shi, Shizuoka 418-0004 (JP)
(74) Representative: TBK
(86) International application number: PCT/JP2019/013336
(87) International publication number: WO 2019/189451

(57) **Abstract**

The prefilled syringe according to the present disclosure includes: a liquid medicine; a barrel including a cylindrical barrel body section that contains the liquid medicine, and a nozzle section that is provided on a distal end side of the barrel body section and that discharges the liquid medicine, the barrel being provided with a proximal end opening on a proximal end section of the barrel body section; a cap that seals a distal end opening provided on a distal end section of the nozzle section; a gasket that slides on an inner circumferential surface of the barrel body section; a syringe plunger that can be mounted to the gasket and has an insertion section that can be inserted into the barrel body section; and an RFID tag mounted on the insertion section and including an antenna for communication and a memory.

## Description

### Technical Field

The present disclosure relates to a prefilled syringe.

### Background Art

When a medicine such as an intravenous anesthetic is delivered into the patient's body in a medical setting such as an operating room or an intensive care unit, it is necessary to deliver the medicine over a long time while adjusting a flow rate (hereinafter also simply referred to as a "liquid delivery amount") of the medicine to be delivered according to a technique to be applied, patient's condition, and the like. As a device for accurately delivering a medicine over a long period of time with a set liquid delivery amount, a liquid medicine administration device, such as a syringe pump, capable of delivering a liquid medicine containing a medicine using a prefilled syringe that contains the liquid medicine in a barrel has been known, for example.

Patent Literature 1 discloses a liquid medicine injection system as a liquid medicine administration device of this type. Patent Literature 1 also discloses a syringe which is to be mounted on the liquid medicine injection system and which has a radio frequency identification (RFID) chip attached thereto, the RFID chip having various kinds of data recorded thereon. Further, the liquid medicine injection system disclosed in Patent Literature 1 has an RFID reader that acquires various kinds of recorded data from the RFID chip of the syringe.

Patent Literature 2 also discloses a syringe which is equipped with an RFID device and which is mounted on a liquid medicine administration device.

### Citation List

### Patent Literature

Patent Literature 1: JP 5802713 B2
Patent Literature 2: US 2015/0217059

### Summary of Invention

### Technical Problem

In the syringe disclosed in Patent Literature 1, the RFID chip is mounted on the outer surface of the barrel of the syringe. Therefore, the RFID chip may be damaged during transportation of the syringe, for example. Further, when the liquid medicine injection system disclosed in Patent Literature 1 is used, the syringe is mounted on a base part of the liquid medicine injection system in a medial setting. When the syringe is mounted on the base part, the RFID chip attached to the outer surface of the barrel may also be damaged.

In order to prevent damage on the RFID chip during transportation or mounting of the syringe, it is conceivable to incorporate the RFID device into a gasket mounted in the syringe, as disclosed in Patent Literature 2. However, when the gasket is mounted in the syringe, the gasket may be largely deformed and the RFID tag may be damaged.

An object of the present disclosure is to provide a prefilled syringe including an RFID tag that is less likely to be damaged.

### Solution to Problem

A prefilled syringe according to a first aspect of the present invention includes: a liquid medicine; a barrel including a barrel body section that is cylindrical and that contains the liquid medicine, and a nozzle section that is provided on a distal end side of the barrel body section and that discharges the liquid medicine, the barrel being provided with a proximal end opening on a proximal end section of the barrel body section; a cap that seals a distal end opening provided on a distal end section of the nozzle section; a gasket that slides on an inner circumferential surface of the barrel body section; a syringe plunger that is mountable to the gasket and has an insertion section insertable into the barrel body section; and an RFID tag mounted on the insertion section of the syringe plunger, the RFID tag including an antenna for communication and a memory.

As one embodiment of the present invention, the syringe plunger includes a mounting member mountable to the gasket, and a pressing member mountable to the mounting member, and the insertion section is provided on the mounting member.

As one embodiment of the present invention, the mounting member is formed with a recess that does not contact the gasket, the pressing member, and the barrel body section, and the RFID tag is disposed in the recess.

As one embodiment of the present invention, the gasket has a mounting recess that is open to a proximal end, the mounting member includes a mounting insertion section to be inserted into the mounting recess, and a main body section located on a proximal end side of the mounting insertion section, and the RFID tag is disposed on an outer surface of the main body section.

As one embodiment of the present invention, the main body section of the mounting member includes: a cylindrical body section; a first flange protruding outward from the cylindrical body section in a radial direction and contactable to an inner circumferential surface of the barrel body section; and a second flange that protrudes outward from the cylindrical body section in the radial direction on a proximal side of the cylindrical body section with respect to the first flange and is contactable to the inner circumferential surface of the barrel body section, and the RFID tag is mounted on an outer circumferential surface of the cylindrical body section.

As one embodiment of the present invention, the gasket is made of rubber or elastomer, and has a mounting recess that is open to a proximal end, the mounting member includes a mounting insertion section to be inserted into the mounting recess, and a main body section located on a proximal end side of the mounting insertion section, and the RFID tag is disposed on an outer circumferential surface of the mounting insertion section so as to face an inner circumferential surface of the mounting recess of the gasket.

As one embodiment of the present invention, the RFID tag includes: a tag body having the antenna and the memory; and a label section that carries the tag body and has an attachment surface attached to an outer surface of the mounting member.

As one embodiment of the present invention, the RFID tag includes: a tag body having the antenna and the memory; and a cover that covers the tag body, the RFID tag being accommodated inside the mounting member.

As one embodiment of the present invention, the RFID tag is formed inside the mounting member by insert molding. Advantageous Effects of Invention

The present disclosure can provide a prefilled syringe including an RFID tag that is less likely to be damaged.

### Brief Description of Drawings

Fig. 1 is a perspective view showing a prefilled syringe mounted to a syringe pump as one embodiment.
Fig. 2 is a perspective view showing the prefilled syringe shown in Fig. 1.
Fig. 3 is a sectional view showing the prefilled syringe shown in Fig. 2.
Fig. 4 is a perspective view showing a mounting member of the prefilled syringe shown in Fig. 2.
Fig. 5 is a block diagram showing an RFID tag mounted to the prefilled syringe shown in Fig. 2 and a reader of the syringe pump.
Fig. 6 is a diagram showing the RFID tag mounted on the prefilled syringe shown in Fig. 2.
Fig. 7 is a perspective view showing a first modification of the mounting member of the prefilled syringe shown in Fig. 2.
Fig. 8(a) is a perspective view showing a second modification of the mounting member of the prefilled syringe shown in Fig. 2, and Fig. 8(b) is a view of the mounting member shown in Fig. 8(a) seen from a distal end side.
Fig. 9 is a perspective view showing a third modification of the mounting member of the prefilled syringe shown in Fig. 2.
Fig. 10 is a side view showing a fourth modification of the mounting member of the prefilled syringe shown in Fig. 2.
Fig. 11(a) is a perspective view showing a fifth modification of the mounting member of the prefilled syringe shown in Fig. 2 as seen from a distal end side, and Fig. 11(b) is a side view of the mounting member shown in Fig. 11(a).
Fig. 12(a) is a view showing a sixth modification of the mounting member of the prefilled syringe shown in Fig. 2 as seen from a distal end side, and Fig. 12(b) is a sectional view along a line I-I in Fig. 12(a).
Fig. 13 is a diagram showing a modification of the RFID tag mounted on the prefilled syringe shown in Fig. 2.
Fig. 14(a) is a side view showing a modification of a syringe plunger of the prefilled syringe shown in Fig. 2, and Fig. 14(b) is a sectional view along a line II-II in Fig. 14(a).
Fig. 15 is a diagram showing another arrangement of the RFID tag on the syringe plunger shown in Fig. 14. Description of Embodiments

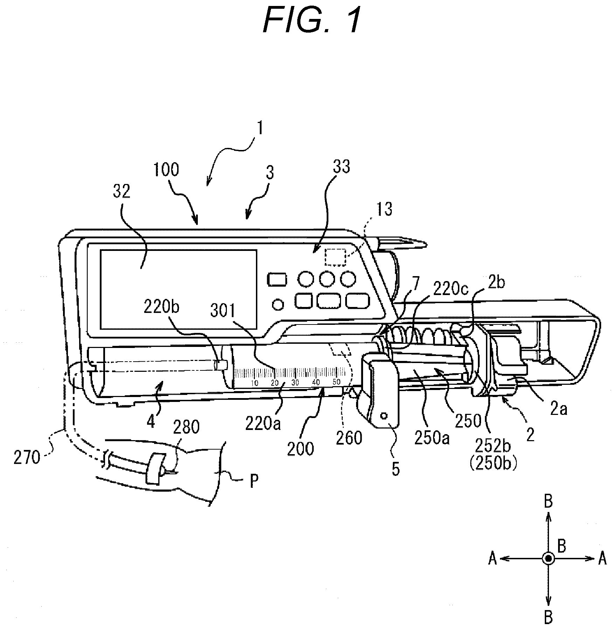

Hereinafter, embodiments of a prefilled syringe according to the present disclosure will be described with reference to Figs. 1 to 15. In the drawings, same members and parts are denoted by the same reference numerals.

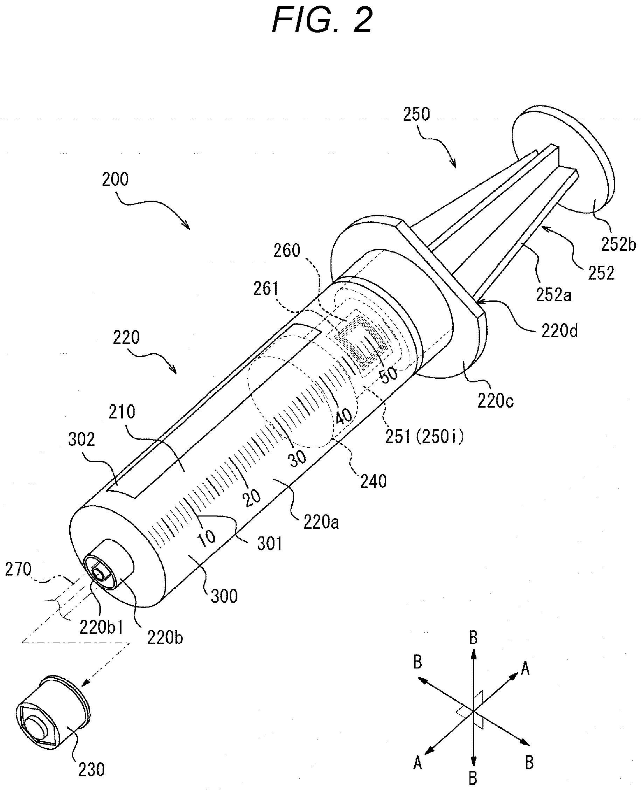

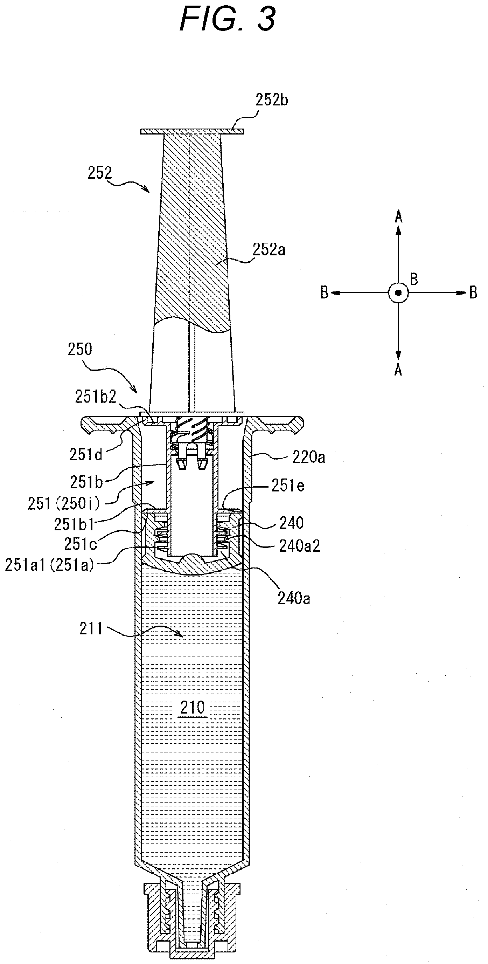

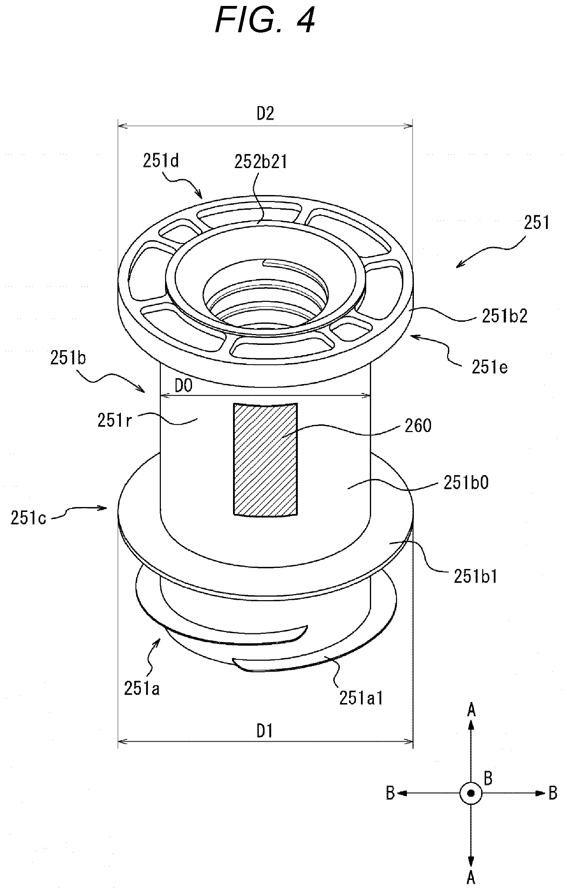

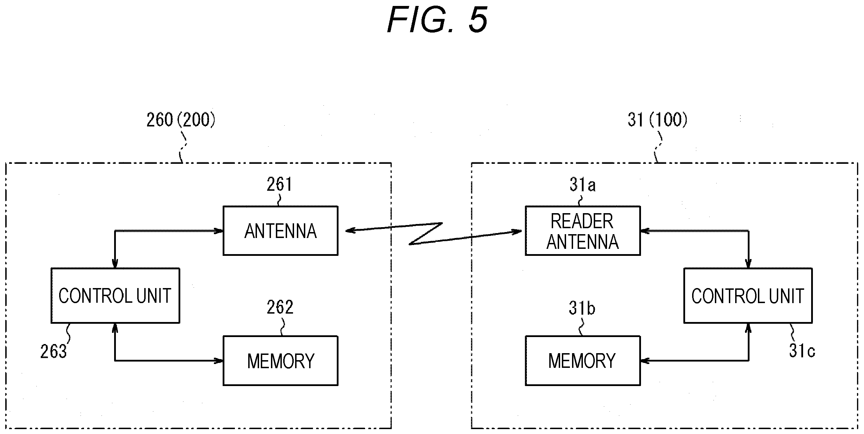

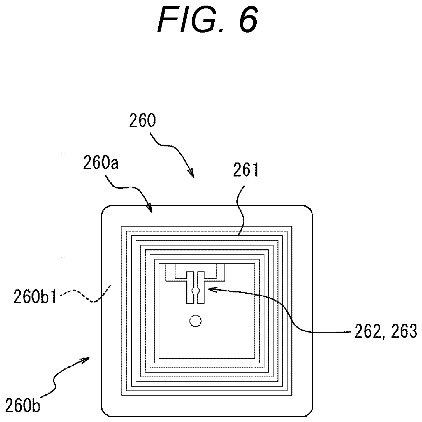

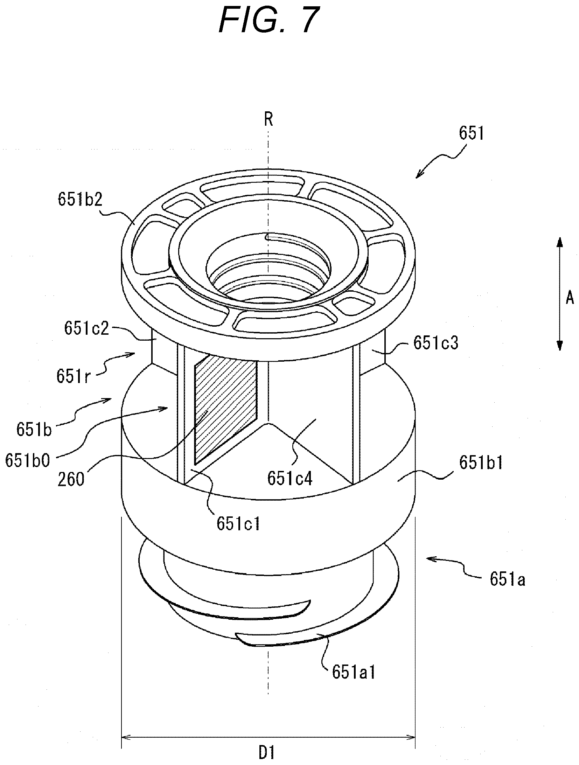

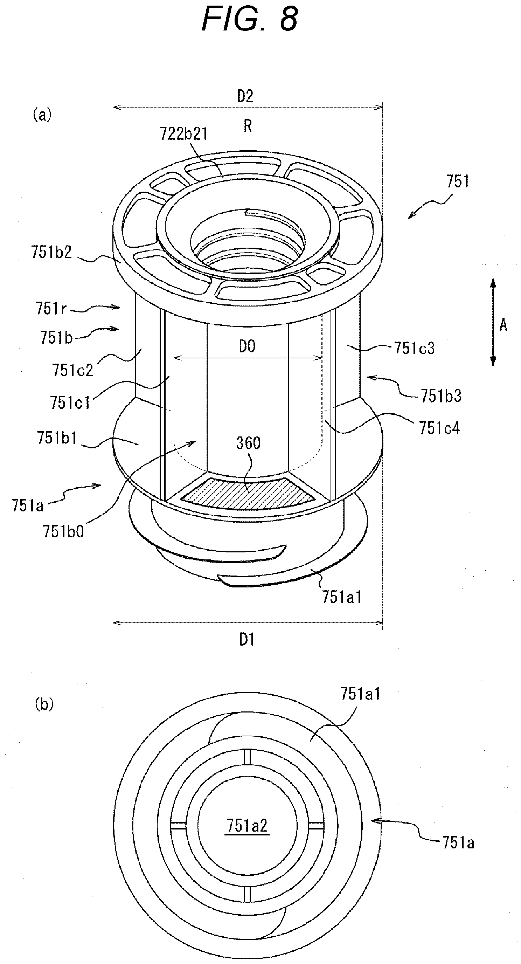

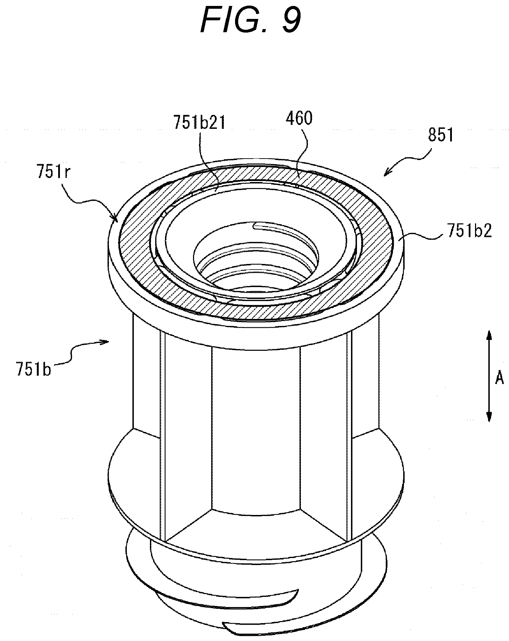

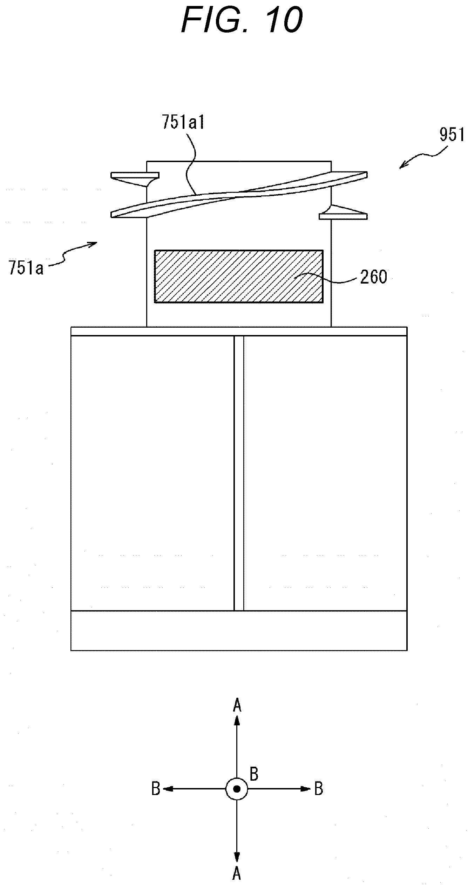

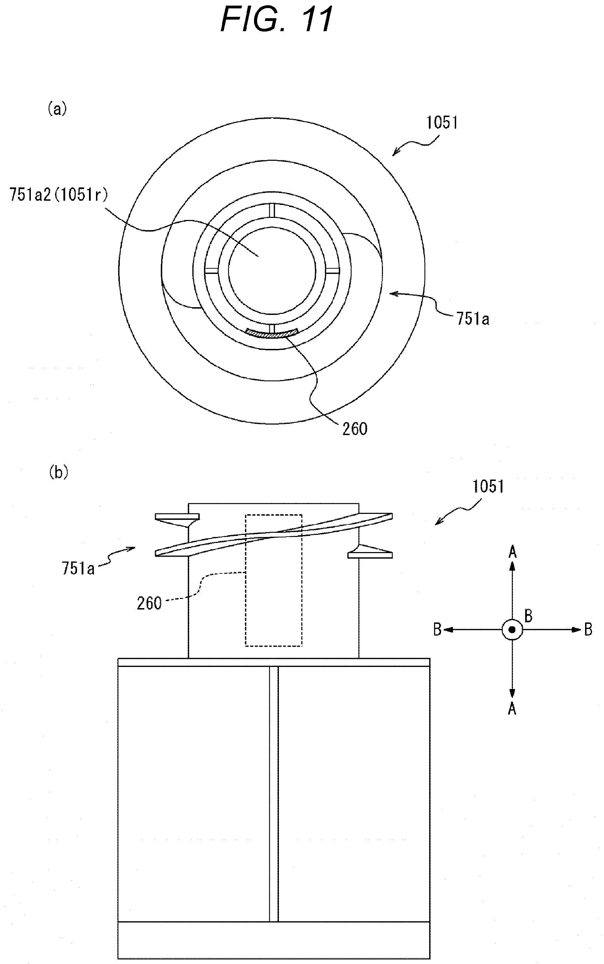

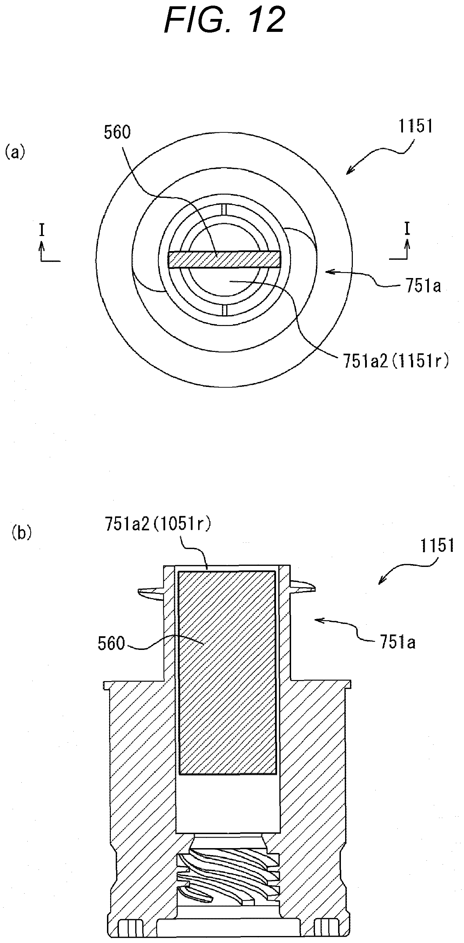

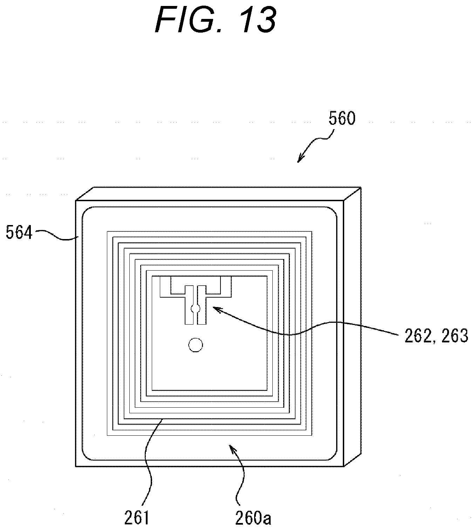

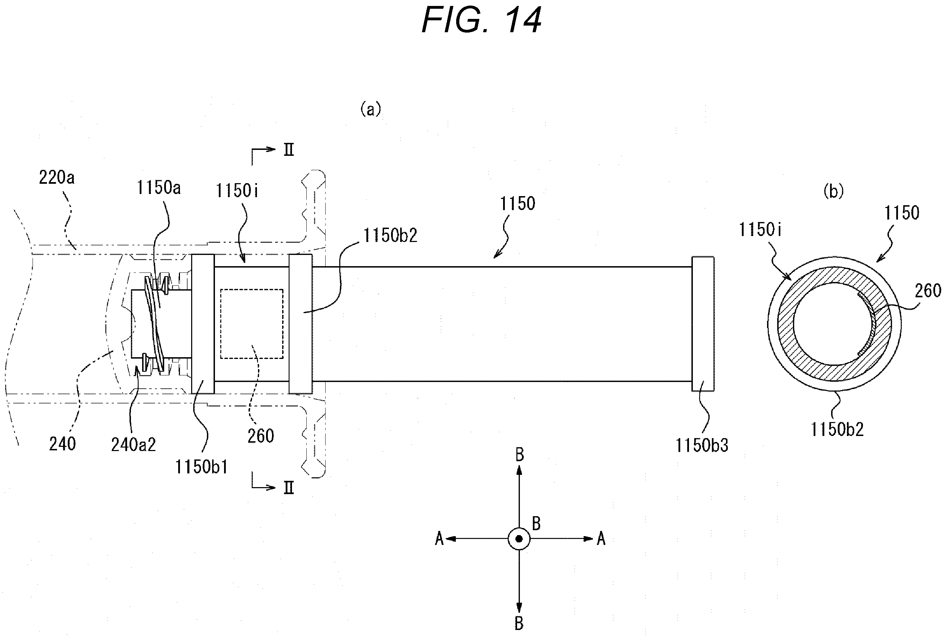

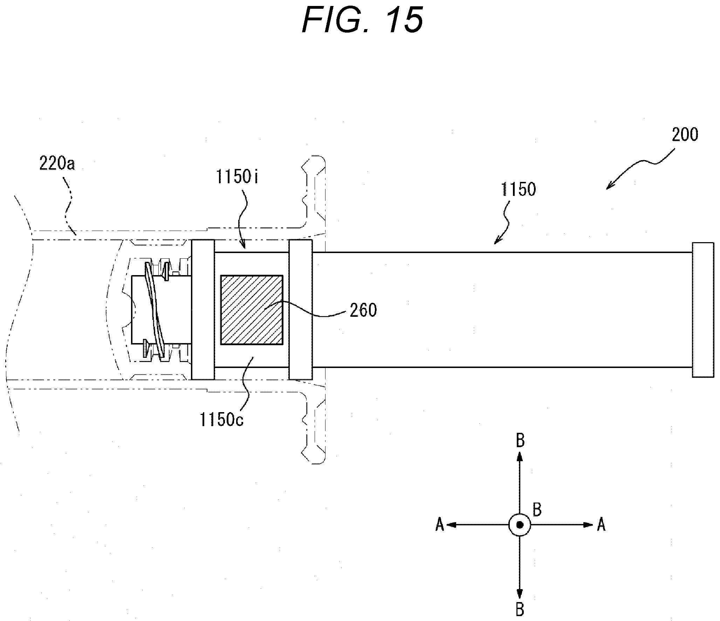

Fig. 1 is a perspective view showing a prefilled syringe 200 mounted to a syringe pump 100 as one embodiment. Fig. 2 is a perspective view showing the prefilled syringe 200 shown in Fig. 1. Fig. 3 is a sectional view showing the prefilled syringe 200 shown in Fig. 2. More specifically, Fig. 3 is a sectional view showing a cross section parallel to the axial direction of the prefilled syringe 200. Fig. 4 is a perspective view showing a mounting member 251 of the prefilled syringe 200 shown in Fig. 2. Fig. 5 is a block diagram showing an RFID tag 260 attached to the prefilled syringe 200 and a reader 31 of the syringe pump 100. Fig. 6 is a diagram showing the RFID tag 260 mounted on the prefilled syringe 200. Fig. 7 is a view showing a mounting member 651 as a first modification of the mounting member 251 of the prefilled syringe 200 shown in Fig. 2. Fig. 8 is a view showing a mounting member 751 as a second modification of the mounting member 251 of the prefilled syringe 200 shown in Fig. 2. Fig. 9 is a view showing a mounting member 851 as a third modification of the mounting member 251 of the prefilled syringe 200 shown in Fig. 2. Fig. 10 is a view showing a mounting member 951 as a fourth modification of the mounting member 251 of the prefilled syringe 200 shown in Fig. 2. Fig. 11 is a view showing a mounting member 1051 as a fifth modification of the mounting member of the prefilled syringe 200 shown in Fig. 2. Fig. 12 is a view showing a mounting member 1151 as a sixth modification of the mounting member 251 of the prefilled syringe 200 shown in Fig. 2. Fig. 13 is a diagram showing an RFID tag 360 as a modification of the RFID tag 260 mounted on the prefilled syringe 200 shown in Fig. 2. Fig. 14 is a view showing a syringe plunger 1150 as a modification of a syringe plunger 250 of the prefilled syringe 200 shown in Fig. 2. Fig. 15 is a diagram showing another arrangement of the RFID tag 260 on the syringe plunger 1150 shown in Fig. 14.

### [Prefilled syringe 200]

As shown in Fig. 1, the prefilled syringe 200 according to the present embodiment can be mounted to the syringe pump 100.

As shown in Fig. 2, the prefilled syringe 200 includes a liquid medicine 210, a barrel 220, a cap 230, a gasket 240, a syringe plunger 250, and an RFID tag 260.

The liquid medicine 210 is, for example, an anticancer agent, an anesthetic, a chemotherapeutic agent, a blood transfusion, a nutrient, or the like. The liquid medicine 210 is contained in the barrel 220. The liquid medicine 210 is injected into the patient's body as described later.

The barrel 220 includes a barrel body section 220a, a nozzle section 220b, and a flange 220c. Further, the barrel 220 is provided with a proximal end opening 220d.

The barrel body section 220a has a tubular shape (for example, a cylindrical shape) and contains the liquid medicine 210 therein.

The nozzle section 220b is provided on the distal end side of the barrel body section 220a. A distal end opening 220b1 is provided at a distal end section of the nozzle section 220b. The liquid medicine 210 is expelled from the distal end opening 220b1. The nozzle section 220b is cylindrical, and a tube 270 (indicated by a chain double-dashed line in Figs. 1 and 2) can be connected to the nozzle section 220b.

The flange 220c protrudes outward from the proximal end section of the barrel body section 220a in the radial direction B of the barrel body section 220a.

The proximal end opening 220d is provided at the proximal end section of the barrel body section 220a. An insertion section 250i of the syringe plunger 250, which will be described later, is inserted through the proximal end opening 220d.

The cap 230 can seal the distal end opening 220b1 provided at the distal end section of the nozzle section 220b. Fig. 2 shows a state in which the cap 230 is removed from the nozzle section 220b, and the distal end opening 220b1 is opened.

The gasket 240 is made of rubber or elastomer in the present embodiment. The gasket 240 can slide on the inner circumferential surface of the barrel body section 220a. The gasket 240 in the present embodiment is a tubular body having a closed distal end and an open proximal end. In other words, the gasket 240 has a mounting recess 240a that is open toward the proximal end and extends along the axial direction A of the barrel body section 220a, as shown in Fig. 3. A mounting member engagement section 240a2 is formed in the mounting recess 240a.

As shown in Fig. 3, the gasket 240 constitutes a liquid medicine containing section 211 in which the liquid medicine 210 is contained in the barrel 220. Note that an amount (ml) of liquid medicine to be contained is associated with the outer diameter D (mm) of the prefilled syringe 200, and is specified in, for example, ISO 11040-6 (Prefilled syringes-Part 6: Plastic barrels for injectables). In the present embodiment, when a prefilled syringe that can contain 5 ml of liquid medicine is used, the outer diameter D is 14 mm. In addition, when the amount of the liquid medicine that can be contained is 10 ml, the outer diameter D is 17 mm. When the amount of the liquid medicine that can be contained is 20 ml, the outer diameter D is 22 mm. Further, when the amount of the liquid medicine that can be contained is 50 ml, the outer diameter D is 33 mm.

As shown in Fig. 3, the syringe plunger 250 can be mounted to the gasket 240. In other words, the syringe plunger 250 has an insertion section 250i that can be inserted into the barrel body section 220a. In the present embodiment, the syringe plunger 250 includes a mounting member 251 that can be mounted to the gasket 240 and a pressing member 252 that can be mounted to the mounting member 251. In the present embodiment, the entire mounting member 251 constitutes the insertion section 250i. As will be described later, the RFID tag 260 is mounted on the insertion section 250i. Note that, in modifications of the mounting member described later (see Fig. 7, and the like), the entire mounting member also constitutes the insertion section 250i, and the RFID tag 260 is also mounted on the insertion section 250i.

As shown in Figs. 3 and 4, the mounting member 251 includes a mounting insertion section 251a that is inserted into the mounting recess 240a of the gasket 240, and a main body section 251b that is located on the proximal end side of the mounting insertion section 251a. In other words, the mounting insertion section 251a is a portion protruding from the distal end of the main body section 251b.

As shown in Fig. 4, a gasket engagement section 251a1 is formed on the outer circumferential surface of the mounting insertion section 251a of the mounting member 251. As shown in Fig. 3, the gasket 240 and the mounting member 251 are engaged with each other by the mounting member engagement section 240a2 of the gasket 240 and the gasket engagement section 251a1 of the mounting member 251. This engagement can be achieved by a thread connection. It is to be noted that, in the present embodiment, after the gasket 240 and the mounting member 251 are engaged with each other, a male screw section as the gasket engagement section 251a1 of the mounting member 251 protrudes further to the distal side beyond a female screw section serving as the mounting member engagement section 240a2 of the gasket 240 as shown in Fig. 3. As a result, the male screw section and the female screw section are not engaged with each other, so that even if the mounting member 251 and the gasket 240 relatively rotate, the thread connection is less likely to loosen, and the mounting member 251 is not easily detached from the gasket 240.

As shown in Fig. 3, the main body section 251b of the mounting member 251 extends along the axial direction A of the barrel body section 220a. The pressing member 252 can be mounted to the main body section 251b of the mounting member 251. As shown in Fig. 4, the main body section 251b has a cylindrical body section 251b0, a first flange 251b1, and a second flange 251b2.

As shown in Fig. 4, the first flange 251b1 protrudes outward from the cylindrical body section 251b0 in the radial direction (hereinafter, referred to as "radial direction B", because it is the same as the radial direction B of the barrel body section 220a in a state where the mounting member 251 is inserted into the barrel body section 220a). Therefore, the outer diameter D1 of the first flange 251b1 is larger than the outer diameter D0 of the cylindrical body section 251b0. In the present embodiment, the first flange 251b1 protrudes from the distal end section of the cylindrical body section 251b0. Further, the first flange 251b1 can contact the inner circumferential surface of the barrel body section 220a. In other words, the outer diameter D1 of the first flange 251b1 is slightly smaller than or substantially equal to the inner diameter of the barrel body section 220a.

The second flange 251b2 protrudes outward from the cylindrical body section 251b0 in the radial direction B on the proximal side of the cylindrical body section 251b0 with respect to the first flange 251b1. The outer diameter D2 of the second flange 251b2 is larger than the outer diameter D0 of the cylindrical body section 251b0. In the present embodiment, the second flange 251b2 protrudes from the proximal end section of the cylindrical body section 251b0. The second flange 251b2 can also contact the inner circumferential surface of the barrel body section 220a. In other words, the outer diameter D2 of the second flange 251b2 is slightly smaller than or substantially equal to the inner diameter of the barrel body section 220a. Further, as shown in Fig. 4, an inner edge 251b21 of the second flange 251b2 protrudes further than the other portions of the second flange 251b2 in the axial direction A.

In the present embodiment, the mounting member 251 is formed with a recess 251r that does not contact the gasket 240, the pressing member 252, and the barrel body section 220a. In other words, the mounting member 251 includes a gasket contact section 251c that can contact the gasket 240, a pressing member contact section 251d that can contact the pressing member 252, and a barrel body contact section 251e that can contact the inner circumferential surface of the barrel body section 220a. The recess 251r is formed in a region different from the gasket contact section 251c, the pressing member contact section 251d, and the barrel body contact section 251e. Accordingly, the recess 251r can be separated from the gasket 240, the pressing member 252, and the barrel body section 220a.

In the present embodiment, when the prefilled syringe 200 is shipped, the mounting member 251 on which the RFID tag 260 is mounted can be fixed to the gasket 240 within the barrel 220 containing the liquid medicine 210, as shown in Fig. 2. The same is applied to the modifications (see Fig. 7, etc.) of the mounting member described later. Thus, the prefilled syringe 200 can be transported while the RFID tag 260 in which information about the liquid medicine 210 is written is accommodated in the barrel 220 containing the liquid medicine 210.

As shown in Fig. 3, the pressing member 252 can be mounted to the mounting member 251. The pressing member 252 is movable in the axial direction A of the barrel body section 220a. The pressing member 252 includes a main body section 252a and a flange 252b.

The distal end section of the main body section 252a can be fixed to the mounting member 251. The pressing member 252 and the mounting member 251 can be fixed to each other by a thread connection. Note that, when the prefilled syringe 200 is shipped, the mounting member 251 fixed to the gasket 240 within the barrel 220 and the pressing member 252 can be separated. The pressing member 252 is generally a long member. Therefore, if the prefilled syringe 200 is shipped with the pressing member 252 being separated from the mounting member 251, the prefilled syringe 200 can be transported with the total length of the prefilled syringe 200 in the axial direction being decreased as compared to the case where the pressing member 252 is not separated from the mounting member 251. A medical staff can fix the pressing member 252 to the mounting member 251 fixed to the gasket 240 within the barrel 220 in a medical setting. It should be noted that the same is applied to the modifications (see Fig. 7, etc.) of the mounting member described later.

The flange 252b protrudes outward from the main body section 252a in the radial direction B on the proximal end section of the pressing member 252.

As shown in Fig. 2, the syringe plunger 250 has the insertion section 250i that can be inserted into the barrel body section 220a. The RFID tag 260 is mounted on the insertion section 250i.

In other words, in the present embodiment, the RFID tag 260 is mounted in the recess 251r that does not contact the gasket 240, the pressing member 252, and the barrel body section 220a as shown in Fig. 4.

Further, in the present embodiment, the shape of the RFID tag 260 is a rectangle as shown in Figs. 2 and 4. The RFID tag 260 is attached to the outer surface of the main body section 251b of the mounting member 251. More specifically, the RFID tag 260 is attached to the outer circumferential surface of the cylindrical body section 251b0 of the main body section 251b as shown in Fig. 4. Further, the RFID tag 260 in the present embodiment is attached to the central part of the cylindrical body section 251b0 of the main body section 251b in the axial direction (hereinafter referred to as "axial direction A", because it is the same as the axial direction A of the barrel body section 220a in a state where the mounting member 251 is inserted into the barrel body section 220a) along the axial direction A. Due to attaching the RFID tag 260 to the outer circumferential surface of the cylindrical body section 251b0, the size of the RFID tag 260 can be sufficiently increased. Since the size of the RFID tag 260 is sufficiently increased, the reader 31 (see Fig. 5) of the syringe pump 100 can more reliably read the data of the RFID tag 260. Note that, when mounted on the syringe pump 100, the RFID tag 260 is set to face the reader 31 of the syringe pump 100. Accordingly, the reader 31 of the syringe pump 100 can more reliably read the data of the RFID tag 260.

As shown in Figs. 5 and 6, the RFID tag 260 has an antenna 261 for communication, a memory 62, and a control unit 263. In the present embodiment, as shown in Fig. 6, the RFID tag 260 can be provided with a tag body 260a having the antenna 261 and the memory 262, and a label section 260b that can carry the tag body 260a and that has an attachment surface 260b1 (back surface in Fig. 6) to be attached to a surface such as the outer surface of the mounting member 251. Here, the tag body 260a can be, for example, a plastic substrate.

As shown in Figs. 2 and 6, the antenna 261 of the RFID tag 260 is structured from an antenna wire wound in a rectangle. The antenna wire is not limited to being wound in a rectangle and may be wound in a circle. However, as shown in Fig. 3, the area on the insertion section 251i where the RFID tag 260 can be mounted is limited. Therefore, it is preferable to wind the antenna wire in a rectangle. This makes it easier to ensure a large loop area formed by the antenna wire, as compared with a configuration in which the antenna wire is wound in a circle. Thus, even if the RFID tag 260 of the prefilled syringe 200 and the reader 31 (see Fig. 5) of the syringe pump 100 are not aligned with high precision when the prefilled syringe 200 is mounted on a supporting section 4 of the syringe pump 100 as shown in Fig. 1, the RFID tag 260 can communicate with the reader.

The antenna 261 of the RFID tag 260 performs communication by wireless communication having a short working distance such as near field communication (NFC).

As shown in Fig. 5, the control unit 263 of the RFID tag 260 can read data from the memory 262 and cause the antenna 261 to transmit the data. In the wireless communication between the antenna 261 of the RFID tag 260 and a reader antenna 31a of the reader 31 of the syringe pump 100, a communicable distance is short (for example, within 35 mm). Therefore, when the RFID tag 260 of the prefilled syringe 200 is distant from the reader antenna 31a of the reader 31 by a predetermined distance or more, the reader antenna 31a of the reader 31 cannot communicate with the RFID tag 260 of the prefilled syringe 200. The memories 262 and 263 of the RFID tag 260 can be constituted by, for example, an integrated circuit (IC chip) including a non-volatile memory.

As shown in Fig. 5, the antenna 261 of the RFID tag 260 receives an electromagnetic wave transmitted from the reader antenna 31a of the reader 31 of the syringe pump 100. The operating power of the RFID tag 260 can be obtained from this electromagnetic wave. The control unit 263 reads the data in the memory 262 of the RFID tag 260, and sends (transmits) the data to the reader antenna 31a of the reader 31 through the electromagnetic wave using the antenna 261. The reader antenna 31a of the reader 31 receives the electromagnetic wave from the antenna 261 of the RFID tag 260. Then, a control unit 31c of the syringe pump 100 acquires the data stored in the memory 262 of the RFID tag 260 by extracting the data from the received electromagnetic wave, and stores the data in a storage unit 31b of the syringe pump 100.

The memory 262 of the RFID tag 260 stores, for example, various kinds of data regarding the prefilled syringe 200, such as the name of the liquid medicine 210, identification data for each prefilled syringe, dimensional data of the barrel 220, and dimensional data of the stroke of the syringe plunger 250.

As shown in Fig. 2, the information label 300 is attached to the outer circumferential surface of the barrel body section 220a.

The information label 300 is provided with two scales 301 which extend along the axial direction A of the barrel body section 220a. More specifically, the notches of the two scales 301 in the present embodiment are arranged along the axial direction A of the barrel body section 220a. The scales 301 indicate an amount of the liquid medicine 210 in the barrel body section 220a. In the present embodiment, when the information label 300 is attached to the outer circumferential surface of the barrel body section 220a as shown in Fig. 2, the two scales 301 are symmetrical with respect to the central axis of the barrel body section 220a.

The information label 300 can be provided with an information area 302 in which the name of the medicine or an amount of the liquid medicine is written, in addition to the scales 301.

### [Syringe pump 100]

Next, the syringe pump 100 will be described with reference to Fig. 1.

The syringe pump 100 is used in, for example, an intensive care unit. Further, the syringe pump 100 can be used when a liquid medicine such as an anticancer agent, an anesthetic, a chemotherapeutic agent, a blood transfusion, or a nutrient is microinjected to a patient P over a relatively long time with high accuracy.

Further, the syringe pump 100 according to the present embodiment can be mounted to and removed from a stand or the like, and can be used while being mounted on the stand or the like. The prefilled syringe 200 is fixed to the syringe pump 100 such that the axial direction A of the barrel body section 220a coincides with the horizontal direction.

As shown in Fig. 1, the syringe pump 100 includes a syringe plunger driving section 2, a main body 3, a supporting section 4, and a clamp section 5.

The syringe plunger driving section 2 drives the syringe plunger 250 of the prefilled syringe 200 in the distal direction toward the distal end of the barrel body section 220a. Note that the syringe plunger driving section 2 can similarly drive a syringe plunger 1150 shown in Figs. 14 and 15.

The syringe plunger driving section 2 in the present embodiment includes a pressing part 2a and a flange fixing part 2b.

The pressing part 2a is located proximal to the flange 252b of the pressing member 252 as the flange 250b of the syringe plunger 250 of the mounted prefilled syringe 200 in the axial direction A. Then, when the pressing part 2a is moved toward the distal side in the axial direction A, the surface of the flange 250b of the syringe plunger 250 on the proximal side in the axial direction A can be pressed toward the distal side in the axial direction A. Accordingly, the syringe plunger 250 can be relatively moved to the distal side in the axial direction A with respect to the barrel 220 of the mounted prefilled syringe 200.

The flange fixing part 2b fixes the flange 250b of the syringe plunger 250 to the pressing part 2a. Specifically, the flange fixing part 2b in the present embodiment is located on the distal side of the pressing part 2a in the axial direction A and is attached to the pressing part 2a. While the prefilled syringe 200 is mounted, the flange 250b of the syringe plunger 250 is located between the pressing part 2a and the flange fixing part 2b. Thus, the syringe plunger 250 is movable in the axial direction A with the movement of the syringe plunger driving section 2 in the axial direction A.

The flange 220c of the barrel 220 is engaged with a flange receiving groove 7 of the main body 3. The main body 3 is provided with the reader 31 including the reader antenna 31a (see Fig. 5) that receives a data set stored in the memory 262 of the RFID tag 260 of the prefilled syringe 200, and a control unit 13 that controls the syringe plunger driving section 2. Specifically, the reader 31 and the control unit 13 in the present embodiment are arranged inside the main body 3. Note that the control unit 13 of the syringe pump 100 and the control unit 31c described above can be provided as separate components. Further, the control unit 31c and the control unit 13 may be integrated as the same component. In the present embodiment, the reader antenna 31a of the reader 31 is structured from a reader antenna wire. The outer perimeter of the reader antenna 31a structured from the reader antenna wire is rectangular.

Referring to Fig. 5, the reader antenna 31a of the reader 31 can emit an electromagnetic wave in a state where the prefilled syringe 200 is received by the later-described supporting section 4. The RFID tag 260 attached to the prefilled syringe 200 transmits data in response to the electromagnetic wave. The reader antenna 31a of the reader 31 can receive the data.

Further, as shown in Fig. 1, the main body 3 in the present embodiment includes a display unit 32 and an operation panel 33.

As shown in Fig. 1, the supporting section 4 in the present embodiment is formed on the front surface of the main body 3. Further, the supporting section 4 in the present embodiment supports the outer circumferential surface of the barrel body section 220a of the prefilled syringe 200 in a direction perpendicular to the central axis of the barrel body section 220a. The supporting section 4 in the present embodiment is constituted by a concave curved surface having a substantially semicircular cross section in order to receive the outer circumferential surface of the barrel body section 220a. Further, the supporting section 4 in the present embodiment can receive a plurality of types of barrel body sections 220a having different sizes such as outer diameters.

As shown in Fig. 1, the clamp section 5 faces the supporting section 4 formed on the main body 3 and clamps the barrel body section 220a of the prefilled syringe 200 with the supporting section 4.

The prefilled syringe 200 operates as follows in the mounted state mounted on the syringe pump 100. The syringe plunger 250 is pressed toward the distal side in the axial direction A by the syringe pump 100. As a result, the gasket 240 (see Fig. 2, etc.) connected to the syringe plunger 250 slides toward the distal side in the axial direction A within the barrel body section 220a of the barrel 220.

When the gasket 240 slides toward the distal side in the axial direction A within the barrel body section 220a, the liquid medicine 210 (see Fig. 2, etc.) in the barrel body section 220a is compressed. The liquid medicine 210 is expelled through the nozzle section 220b of the barrel body section 220a by the compressive force. When the syringe pump 100 is used, the tube 270 is connected to the distal end opening 220b1 (see Fig. 2, etc.) of the nozzle section 220b of the prefilled syringe 200. Further, as shown in Fig. 1, an indwelling needle 280 to be indwelled in the patient P is connected to the distal end of the tube 270. Therefore, the liquid medicine 210 in the barrel body section 220a can be delivered into the body of the patient P through the tube 270 and the indwelling needle 280.

### [Mounting member 651]

Fig. 7 shows a mounting member 651 as a first modification of the mounting member 251 described above. The mounting member 651 can be mounted to the gasket 240 (see Fig. 3).

As shown in Fig. 7, the mounting member 651 includes a mounting insertion section 651a to be inserted into the mounting recess 240a (see Fig. 3, etc.) of the gasket 240 (see Fig. 3, etc.), and a main body section 651b located on the proximal end side of the mounting insertion section 651a.

As shown in Fig. 7, a gasket engagement section 651a1 is formed on the outer circumferential surface of the mounting insertion section 651a of the mounting member 651. The gasket 240 and the mounting member 651 are engaged with each other by the mounting member engagement section 240a2 (see Fig. 3, etc.) of the gasket 240 and the gasket engagement section 651a1 of the mounting member 651. This engagement can be achieved by a thread connection.

The main body section 651b of the mounting member 651 extends along the axial direction A of the barrel body section 220a. The pressing member 252 (see Fig. 3) can be mounted on the main body section 651b of the mounting member 651. As shown in Fig. 7, the main body section 651b has a plate-shaped main body 651b0, a first flange 651b1, and a second flange 651b2.

The plate-shaped main body 651b0 has four plates 651c1 to 651c4 extending radially from a central axis R of the mounting member 651. In other words, in the cross-sectional view of the plate-shaped main body 651b0 orthogonal to the central axis R of the mounting member 651, four plates 651c1 to 651c4 are provided in a cross shape. In the present embodiment, the RFID tag 260 is arranged on one surface of one plate 651c1.

The first flange 651b1 in Fig. 7 is a disk-shaped portion that is located at the distal end section of the main body section 651b and has the central axis R of the mounting member 651 as its axis. The outer circumferential surface defining the outer diameter D1 of the first flange 651b1 is located at a position same as or outside of the outer edge of the plate-shaped main body 651b0 in the radial direction with respect to the central axis R. The thickness of the first flange 651b1 along the axial direction A is larger than that of the first flange 251b1 of the mounting member 251 shown in Fig. 4. Accordingly, the rigidity of the first flange 651b1 can be set higher than the rigidity of the first flange 251b1. Further, the first flange 651b1 can contact the inner circumferential surface of the barrel body section 220a.

The second flange 651b2 in Fig. 7 is a disk-shaped portion that is located at the proximal end section of the main body section 651b and has the central axis R of the mounting member 651 as its axis. The outer circumferential surface defining the outer diameter D2 of the second flange 651b2 is located at a position same as or outside of the outer edge of the plate-shaped main body 651b0 in the radial direction with respect to the central axis R. The thickness of the second flange 651b2 along the axial direction A is the same as that of the second flange 251b2 of the mounting member 251 shown in Fig. 4. Further, the second flange 651b2 can contact the inner circumferential surface of the barrel body section 220a.

In the present embodiment, the mounting member 651 is formed with a recess 651r that does not contact the gasket 240 (see Fig. 2, etc.), the pressing member 252 (see Fig. 2, etc.), and the barrel body section 220a (see Fig. 2, etc.).

In the present embodiment, the RFID tag 260 is arranged in the recess 651r that does not contact the gasket 240, the pressing member 252, and the barrel body section 220a as shown in Fig. 7.

In the present modification, the shape of the RFID tag 260 is a rectangle as shown in Fig. 7. The RFID tag 260 is attached to the outer surface of the main body section 651b of the mounting member 651. More specifically, the RFID tag 260 is arranged on one side of one plate 651c1. The RFID tag 260 is attached on almost the entire surface of at least one surface of the plate 651c1, whereby the RFID tag 260 can be sufficiently increased so that the reader 31 (see Fig. 5) of the syringe pump 100 can easily read the data of the RFID tag 260. Further, since the RFID tag 260 in Fig. 7 is arranged on the flat plate 651c1, it does not deform like the RFID tag 260 arranged on the cylindrical surface shown in Fig. 4. Therefore, the RFID tag 260 shown in Fig. 7 is less likely to be damaged than the RFID tag 260 shown in Fig. 4.

### [Mounting member 751]

Fig. 8 shows a mounting member 751 as a second modification of the mounting member 251 described above. The mounting member 751 can be mounted on the gasket 240 (see Fig. 3).

As shown in Fig. 8, the mounting member 751 includes a mounting insertion section 751a to be inserted into the mounting recess 240a (see Fig. 3, etc.) of the gasket 240 (see Fig. 3, etc.), and a main body section 751b located on the proximal end side of the mounting insertion section 751a.

As shown in Fig. 8, a gasket engagement section 751a1 is formed on the outer circumferential surface of the mounting insertion section 751a of the mounting member 751. The gasket 240 and the mounting member 751 are engaged with each other by the mounting member engagement section 240a2 (see Fig. 3, etc.) of the gasket 240 and the gasket engagement section 751a1 of the mounting member 751. This engagement can be achieved by a thread connection.

As shown in Fig. 8(a), the mounting insertion section 751a has a cylindrical shape and has a hole 751a2.

As shown in Fig. 8, the main body section 751b of the mounting member 751 extends along the axial direction A of the barrel body section 220a. The pressing member 252 (see Fig. 3) can be mounted on the main body section 751b of the mounting member 751. As shown in Fig. 8, the main body section 751b has a cylindrical body section 751b0, a plate-shaped section 751b3, a first flange 751b1, and a second flange 751b2.

The cylindrical body section 751b0 is a cylinder extending along the axial direction A and having an outer diameter D0.

The plate-shaped section 751b3 has four plates 751c1 to 751c4 extending radially (in a cross shape) from the cylindrical body section 751b0 in a cross section orthogonal to the central axis R of the mounting member 651.

The first flange 751b1 protrudes outward from the cylindrical body section 751b0 in the radial direction, and has an outer diameter D1 larger than the outer diameter D0 of the cylindrical body section 751b0. In the present embodiment, the first flange 751b1 protrudes from the distal end section of the cylindrical body section 751b0. Further, the first flange 751b1 can contact the inner circumferential surface of the barrel body section 220a (see Fig. 2, etc.). In the present embodiment, an RFID tag 360 is mounted in one of four regions that are sectioned by the four plates 751c1 to 751c4 on the proximal end side of the first flange 751b1.

The second flange 751b2 protrudes outward from the cylindrical body section 751b0 in the radial direction on the proximal side of the cylindrical body section 751b0 with respect to the first flange 751b1, and has an outer diameter D2 larger than the outer diameter D0 of the cylindrical body section 751b0. In the present embodiment, the second flange 751b2 protrudes from the proximal end section of the cylindrical body section 751b0. The second flange 751b2 can also contact the inner circumferential surface of the barrel body section 220a. Further, an inner edge 751b21 of the second flange 751b2 protrudes further than the other portions of the second flange 751b2 in the axial direction A.

In the present embodiment, the mounting member 751 is formed with a recess 751r that does not contact the gasket 240 (see Fig. 2, etc.), the pressing member 252 (see Fig. 2, etc.), and the barrel body section 220a (see Fig. 2, etc.). In other words, the recess 751r is a region defined by the outer surface of the cylindrical body section 751b0 and both surfaces of the respective four plates 751c1 to 751c4. Regarding the recess, the same applies to the modifications described later.

In the present embodiment, the RFID tag 360 is arranged in the recess 751r that does not contact the gasket 240, the pressing member 252, and the barrel body section 220a as shown in Fig. 8.

In the present modification, the RFID tag 360 has a curved band shape as shown in Fig. 8. Accordingly, the size of the RFID tag 360 can be increased so that the reader of the syringe pump 100 can easily read the data of the RFID tag 360. Further, the antenna of the RFID tag 360 can be formed by winding a conductive wire so that the outer perimeter has an arc shape. Note that the other configuration of the RFID tag 360 is the same as the configuration of the RFID tag 260 described above.

The RFID tag 360 is attached to the outer surface of the main body section 751b of the mounting member 751. More specifically, the RFID tag 360 is mounted in almost the entire surface on the proximal end side of the first flange 751b1 in one of the four regions that are sectioned by the four plates 751c1 to 751c4. Accordingly, the size of the RFID tag 360 can be sufficiently increased so that the reader of the syringe pump 100 can easily read the data of the RFID tag 360. Further, the RFID tag 360 is arranged on the first flange 751b1 which is flat, whereby the deformation of the RFID tag 360 can be suppressed, and damage of the RFID tag 360 can be prevented.

In another embodiment, the RFID tag 360 can be mounted on the second flange 751b2 instead of the first flange 751b1. More specifically, the RFID tag 360 is mounted in almost the entire surface (for example, a region facing the region where the RFID tag 260 is mounted in Fig. 8) on the distal end side of the second flange 751b2 in one of the four regions that are sectioned by the four plates 751c1 to 751c4.

### [Mounting member 851]

Fig. 9 shows a mounting member 851 as a third modification of the mounting member 251 described above. The mounting member 851 can be mounted on the gasket 240 (see Fig. 3).

The mounting member 851 shown in Fig. 9 basically has the same configuration as the mounting member 751 shown in Fig. 8. Therefore, the same members as those of the mounting member 751 in Fig. 8 are designated by the same reference numerals. Hereinafter, a configuration different from the mounting member 751 in Fig. 8 will be mainly described.

A second flange 751b2 is formed at the proximal end section of the mounting member 851. An inner edge 751b21 of the second flange 751b2 protrudes further than the other portions of the second flange 751b2 in the axial direction A. The other portions include a recess 751r that does not contact the gasket 240 (see Fig. 2, etc.), the pressing member 252 (see Fig. 2, etc.) and the barrel body section 220a (see Fig. 2, etc.). An annular groove may be formed on the surface of the second flange 751b2 on the proximal end side, and this groove may be defined as the recess 751r.

In the present modification, an RFID tag 460 is mounted in the recess 751r as shown in Fig. 9. Further, the RFID tag 460 is attached to the outer surface of the main body section 751b of the mounting member 851.

As shown in Fig. 9, the RFID tag 460 preferably has a ring shape. Accordingly, the size of the RFID tag 460 mounted on the annular second flange 751b2 can be increased so that the reader 31 (see Fig. 5) of the syringe pump 100 can easily read the data of the RFID tag 460. Further, the antenna of the RFID tag 460 can be formed by winding a conductive wire so that the outer perimeter has a ring shape. Note that the other configuration of the RFID tag 460 is the same as the configuration of the RFID tag 260 described above.

### [Mounting member 951]

Fig. 10 shows a mounting member 951 as a fourth modification of the mounting member 251 described above. The mounting member 951 can be mounted on the gasket 240 (see Fig. 3).

The mounting member 951 shown in Fig. 10 basically has the same configuration as the mounting member 751 shown in Fig. 8. Therefore, the same members as those of the mounting member 751 in Fig. 8 are designated by the same reference numerals. Hereinafter, a configuration different from the mounting member 751 in Fig. 8 will be mainly described.

With reference to Fig. 10, the RFID tag 260 attached to the mounting member 951 has a rectangular shape. The RFID tag 260 is mounted on the outer circumferential surface of the mounting insertion section 751a on the side proximal to the gasket engagement section 751a1. Further, the mounting insertion section 751a of the mounting member 951 in Fig. 10 is inserted into the mounting recess 240a of the gasket 240 made of rubber or elastomer. That is, the RFID tag 260 shown in Fig. 10 faces the inner circumferential surface of the mounting recess 240a of the gasket 240. Due to the configuration in which the RFID tag 260 is disposed to face the inner circumferential surface of the mounting recess 240a of the gasket 240 which is made of rubber or elastomer having elasticity, the RFID tag 260 can be prevented from being damaged when, for example, an impact is applied to the prefilled syringe 200.

### [Mounting member 1051]

Fig. 11 shows a mounting member 1051 as a fifth modification of the mounting member 251 described above. The mounting member 1051 can be mounted on the gasket 240 (see Fig. 3).

The mounting member 1051 shown in Fig. 11 basically has the same configuration as the mounting member 751 shown in Fig. 8. Therefore, the same members as those of the mounting member 751 in Fig. 8 are designated by the same reference numerals. Hereinafter, a configuration different from the mounting member 751 in Fig. 8 will be mainly described.

As shown in Fig. 11(a), the mounting insertion section 751a of the mounting member 1051 has a cylindrical shape and has a hole 751a2. In other words, the mounting member 1051 is formed with a recess 1051r that is a region not contacting the gasket 240 (see Fig. 2, etc.), the pressing member 252 (see Fig. 2, etc.), and the barrel body section 220a (see Fig. 2, etc.). The rectangular RFID tag 260 is arranged in the recess 1051r. More specifically, the RFID tag 260 is mounted on the inner circumferential surface of the hole 751a2 along the axial direction A. Due to the configuration in which the RFID tag 260 is mounted on the inner circumferential surface of the hole 751a2 of the mounting insertion section 751a, the RFID tag 260 can be protected by the mounting insertion section 751a, whereby the RFID tag 260 can further be prevented from being damaged.

### [Mounting member 1151]

Fig. 12 shows a mounting member 1151 as a sixth modification of the mounting member 251 described above. The mounting member 1151 can be mounted on the gasket 240 (see Fig. 3).

The mounting member 1151 shown in Fig. 12 basically has the same configuration as the mounting member 751 shown in Fig. 8. Therefore, the same members as those of the mounting member 751 in Fig. 8 are designated by the same reference numerals. Hereinafter, a configuration different from the mounting member 751 in Fig. 8 will be mainly described.

First, an RFID tag 560 included in the mounting member 1151 will be described. As shown in Fig. 13, the RFID tag 560 includes a tag body 260a having an antenna 261 and a memory 262, and a cover 564 that covers the tag body 260a.

Here, the mounting insertion section 751a of the mounting member 1151 has a cylindrical shape and has a hole 751a2. In other words, the mounting member 1151 is formed with a recess 1151r that is a region not contacting the gasket 240 (see Fig. 2, etc.), the pressing member 252 (see Fig. 2, etc.), and the barrel body section 220a (see Fig. 2, etc.). The RFID tag 560 is mounted in the recess 1151r. Specifically, the RFID tag 560 is fitted in the hole 751a2, and thus, the RFID tag 560 is accommodated and fixed in the hole 751a2.

According to the prefilled syringe including the mounting member 1151 according to the present modification, the cover 564 protects the tag body 260a, and the RFID tag 560 is prevented from contacting the gasket 240, the pressing member 252, the barrel body section 220a, or the like. Thus, the RFID tag 560 can be prevented from being damaged.

As another example, the RFID tag 560 can be formed in the mounting member 1151 by insert molding. Specifically, the tag body 260a is placed inside the cover 564 formed as a case, the cover 564 is placed in a mold for forming the mounting member 1151, and then resin is injected into the mold. Thus, the mounting member 1151 and the RFID tag 560 can be integrated. Further, the tag body 260a having the antenna 261 and the memory 262 may be embedded in the mounting member 1151. Due to the configuration in which the mounting member 1151 and the RFID tag are integrated, the RFID tag can be protected by the mounting member 1151, whereby damage to the RFID tag can be further suppressed. This configuration also makes it difficult to intentionally remove the RFID tag from the syringe.

### [Syringe plunger 1150]

Fig. 14 shows a syringe plunger 1150 as a modification of the syringe plunger 250 described above.

The syringe plunger 1150 is a single member, unlike the syringe plunger 250 having the mounting member 251 and the pressing member 252 which can be separated from each other shown in Fig. 3. The syringe plunger 1150 has, at the distal end section, an insertion section 1150i that can be inserted into the barrel body section 220a.

The syringe plunger 1150 can be mounted to the gasket 240. More specifically, a gasket engagement section 1150a that engages with the mounting member engagement section 240a2 of the gasket 240 shown in Fig. 3 is formed on the outer circumferential surface of the distal end section of the syringe plunger 1150. This engagement can be achieved by a thread connection.

The insertion section 1150i is provided with a first flange 1150b1 and a second flange 1150b2 that protrude in the radial direction B and can contact the inner circumferential surface of the barrel body section 220a. A third flange 1150b3 protruding in the radial direction B is formed at the proximal end section of the syringe plunger 1150.

The RFID tag 260 is mounted on the insertion section 1150i. More specifically, referring to Fig. 14(b) which is a sectional view taken along a line II-II in Fig. 14(a), the RFID tag 260 is attached to the inner circumferential surface of the syringe plunger 1150 between the first flange 1150b1 and the second flange 1150b2. According to this configuration, the RFID tag 260 is covered with the insertion section 1150i of the syringe plunger 1150, whereby the RFID tag 260 can be strongly protected, and damage to the RFID tag 260 can be suppressed.

In another embodiment, the RFID tag 260 is attached to the outer circumferential surface 1150c of the syringe plunger 1150 between the first flange 1150b1 and the second flange 1150b2 as shown in Fig. 15. According to this configuration, when the prefilled syringe 200 is mounted on the syringe pump 100, the reader of the syringe pump 100 can more reliably read the data of the RFID tag 260. Further, since the RFID tag 260 is mounted on the outer circumferential surface 1150c which is a region not contacting the gasket 240, the pressing member 252, and the barrel body section 220a, damage to the RFID tag 260 can be suppressed.

In the prefilled syringe 200 according to the embodiment of the present disclosure, the syringe plunger 250 has the insertion section 250i that can be inserted into the barrel body section 220a, and the RFID tag 260 is mounted on the insertion section 250i of the syringe plunger 250. As a result, the RFID tag 260 is not exposed to the outer surface of the barrel 220, and thus, damage of the RFID tag that occurs during, for example, transportation and mounting of the prefilled syringe 200 can be prevented. Further, since the RFID tag 260 is not incorporated in the gasket, there is no chance that the RFID tag is damaged when the gasket is mounted in the syringe.

In the prefilled syringe 200 according to the embodiment of the present disclosure, the syringe plunger 250 or 1150 includes the mounting member (251, etc., hereinafter described as 251) that can be mounted to the gasket 240, and the pressing member 252 that can be mounted to the mounting member 251, and the insertion section 250i is provided on the mounting member 251. With this configuration, by fixing the mounting member 251 having the RFID tag (260, etc., hereinafter described as 260) attached thereon to the gasket 240 within the barrel 220 containing the liquid medicine 210, it is possible to prevent the RFID tag 260 into which the information about the liquid medicine 210 is written from being separated from the barrel 220 containing the liquid medicine 210 when the prefilled syringe 200 is used in a medical setting. In addition, when the prefilled syringe 200 is shipped, the mounting member 251 fixed to the gasket 240 within the barrel 220 and the pressing member 252 can be separated. The pressing member 252 is generally a long member. Therefore, separating the mounting member 251 from the pressing member 252 can make the prefilled syringe 200 compact. A medical staff can fix the pressing member 252 to the mounting member 251 fixed to the gasket 240 within the barrel 220 in a medical setting.

In the prefilled syringe 200 according to the embodiment of the present disclosure, the mounting member 251 is formed with the recess 251r that does not contact the gasket 240, the pressing member 252, and the barrel body section 220a, and the RFID tag 260 is disposed in the recess 251r. This can prevent the RFID tag 260 from being damaged due to contact with the gasket 240, the pressing member 252, or the barrel body section 220a during, for example, transportation or mounting of the prefilled syringe 200.

In the prefilled syringe 200 according to the embodiment of the present disclosure, the gasket 240 has the mounting recess 240a that is open to a proximal end, the mounting member 251 includes the mounting insertion section 251a to be inserted into the mounting recess 240a, and the main body section 251b located on a proximal end side of the mounting insertion section 251a, and the RFID tag 260 is disposed on the outer surface of the main body section 251b. With this configuration, the gasket 240 does not contact the RFID tag 260, so that the RFID 260 tag can be prevented from being damaged when the gasket 240 is mounted in the prefilled syringe 200.

In the prefilled syringe 200 according to the embodiment of the present disclosure, the main body section 251b of the mounting member 251 includes: the cylindrical body section 251b0; the first flange 251b1 that protrudes outward from the cylindrical body section 251b0 in the radial direction and that can contact the inner circumferential surface of the barrel body section 220a; and a second flange 251b2 that protrudes outward from the cylindrical body section 251b0 in the radial direction on the proximal side of the cylindrical body section 251b0 with respect to the first flange 251b1 and can contact the inner circumferential surface of the barrel body section 220a, and the RFID tag 260 is mounted on the outer circumferential surface of the cylindrical body section 251b0. This configuration further prevents the RFID tag from contacting the inner circumferential surface of the barrel body section 220a, thereby being capable of preventing the RFID tag 260 from being damaged.

In the prefilled syringe 200 according to the embodiment of the present disclosure, the gasket 240 is made of rubber or elastomer, and has the mounting recess 240a that is open to a proximal end, the mounting member 251 includes the mounting insertion section 251a to be inserted into the mounting recess 240a, and the main body section 251b located on a proximal end side of the mounting insertion section 251a, and the RFID tag 260 is disposed on the outer circumferential surface of the mounting insertion section 251a so as to face the inner circumferential surface of the mounting recess 240a of the gasket 240. Due to the configuration in which the RFID tag 260 is disposed to face the inner circumferential surface of the mounting recess 240a of the gasket 240 which is made of rubber or elastomer having elasticity, the RFID tag 260 can be prevented from being damaged when, for example, an impact is applied to the prefilled syringe 200.

In the prefilled syringe 200 according to the embodiment of the present disclosure, the RFID tag 260 includes: the tag body 260a having the antenna 261 and the memory 262; and the label section 260b that carries the tag body 260a and has the attachment surface 260b1 attached to the outer surface of the mounting member 251. Accordingly, when the prefilled syringe 200 is manufactured, the RFID tag 260 can be easily attached to the insertion section 250i of the syringe plunger 250.

In the prefilled syringe 200 according to the embodiment of the present disclosure, the RFID tag 560 includes: the tag body 260a having the antenna 261 and the memory 262; and the cover 564 that covers the tag body 260a, the RFID tag 560 being accommodated inside the mounting member 251. With this configuration, the cover 564 protects the tag body 260a, and the mounting member 251 protects the RFID tag 560, whereby damage to the RFID tag 560 can be suppressed.

In the prefilled syringe 200 according to the embodiment of the present disclosure, the RFID tag 560 is formed inside the mounting member 251 by insert molding. Accordingly, damage to the RFID tag 560 can be further suppressed. Further, this configuration can make it difficult to intentionally remove the RFID tag 560 from the prefilled syringe 200.

### Reference Signs List

- 100: Syringe pump
- 13: Control unit
- 2: Syringe plunger driving section
- 200: Prefilled syringe
- 210: Liquid medicine
- 211: Liquid medicine accommodating section
- 220: Barrel
- 220a: Barrel body section
- 220b: Nozzle section
- 220b1: Distal end opening
- 220c: Flange
- 220d: Proximal end opening
- 230: Cap
- 240: Gasket
- 240a: Mounting recess
- 240a2: Mounting member engagement section
- 250: Syringe plunger
- 250b: Flange
- 250i: Insertion section
- 251: Mounting member
- 251a: Mounting insertion section
- 251a1: Gasket engagement section
- 251b: Main body section
- 251b0: Cylindrical body section
- 251b1: First flange
- 251b2: Second flange
- 251b21: Inner edge of second flange
- 251c: Gasket contact section
- 251d: Pressing member contact section
- 251e: Barrel contact section
- 251i: Insertion section
- 251r: Recess
- 252: Pressing member
- 252a: Main body section
- 252b: Flange
- 260: RFID tag
- 260a: Tag body
- 260b: Label section
- 260b1: Attachment surface
- 261: Antenna
- 262: Memory
- 263: Control unit
- 270: Tube
- 280: Indwelling needle
- 2a: Pressing part
- 2b: Flange fixing part
- 3: Main body
- 300: Information label
- 301: Scale
- 302: Information area
- 31: Reader
- 31a: Reader antenna
- 31b: Storage unit
- 31c: Control unit
- 32: Display unit
- 33: Operation panel
- 360: RFID tag
- 564: Cover
- 4: Supporting section
- 460: RFID tag
- 5: Clamp section
- 560: RFID tag
- 564: Cover
- 62: Memory
- 651: Mounting member
- 651a: Mounting insertion section
- 651a1: Gasket engagement section
- 651b: Main body section
- 651b0: Plate-shaped main body
- 651b1: First flange
- 651b2: Second flange
- 651c1: Plate
- 651r: Recess
- 7: Groove
- 751: Mounting member
- 751a: Mounting insertion section
- 751a1: Gasket engagement section
- 751a2: Hole
- 751b: Main body section
- 751b0: Cylindrical body section
- 751b1: First flange
- 751b2: Second flange
- 751b21: Inner edge of second flange
- 751b3: Plate-shaped section
- 751c1: Plate
- 751r: Recess
- 851: Mounting member
- 951: Mounting member
- 1051: Mounting member
- 1051r: Recess
- 1150: Syringe plunger
- 1150a: Gasket engagement section
- 1150b1: First flange
- 1150b2: Second flange
- 1150b3: Third flange
- 1150c: Outer circumferential surface
- 1150i: Insertion section
- 1151r: Recess
- 1151: Mounting member
- A: Axial direction of barrel body section
- B: Radial direction of barrel body section
- D: Outer diameter
- DO: Outer diameter
- D1: Outer diameter
- D2: Outer diameter
- P: Patient
- R: Central axis

## Claims

1. A prefilled syringe comprising:
a liquid medicine;
a barrel including a barrel body section that is cylindrical and that contains the liquid medicine, and a nozzle section that is provided on a distal end side of the barrel body section and that discharges the liquid medicine, the barrel being provided with a proximal end opening on a proximal end section of the barrel body section;
a cap that seals a distal end opening provided on a distal end section of the nozzle section;
a gasket that slides on an inner circumferential surface of the barrel body section;
a syringe plunger that is mountable to the gasket and has an insertion section insertable into the barrel body section; and
an RFID tag mounted on the insertion section of the syringe plunger, the RFID tag including an antenna for communication and a memory.

2. The prefilled syringe according to claim 1,
wherein the syringe plunger includes a mounting member mountable to the gasket, and a pressing member mountable to the mounting member, and
the insertion section is provided on the mounting member.

3. The prefilled syringe according to claim 2,
wherein the mounting member is formed with a recess that does not contact the gasket, the pressing member, and the barrel body section, and
the RFID tag is disposed in the recess.

4. The prefilled syringe according to claim 2 or 3,
wherein the gasket has a mounting recess that is open to a proximal end,
the mounting member includes a mounting insertion section to be inserted into the mounting recess, and a main body section located on a proximal end side of the mounting insertion section, and
the RFID tag is disposed on an outer surface of the main body section.

5. The prefilled syringe according to claim 4,
wherein the main body section of the mounting member includes:
a cylindrical body section;
a first flange protruding outward from the cylindrical body section in a radial direction and contactable to an inner circumferential surface of the barrel body section; and
a second flange that protrudes outward from the cylindrical body section in the radial direction on a proximal side of the cylindrical body section with respect to the first flange and is contactable to the inner circumferential surface of the barrel body section, and
the RFID tag is mounted on an outer circumferential surface of the cylindrical body section.

6. The prefilled syringe according to claim 2,
wherein the gasket is made of rubber or elastomer, and has a mounting recess that is open to a proximal end,
the mounting member includes a mounting insertion section to be inserted into the mounting recess, and a main body section located on a proximal end side of the mounting insertion section, and
the RFID tag is disposed on an outer circumferential surface of the mounting insertion section so as to face an inner circumferential surface of the mounting recess of the gasket.

7. The prefilled syringe according to any one of claims 2 to 6,
wherein the RFID tag includes:
a tag body having the antenna and the memory; and
a label section that carries the tag body and has an attachment surface attached to an outer surface of the mounting member.

8. The prefilled syringe according to claim 2 or 3,
wherein the RFID tag includes:
a tag body having the antenna and the memory; and
a cover that covers the tag body,
the RFID tag being accommodated inside the mounting member.

9. The prefilled syringe according to claim 8, wherein the RFID tag is formed inside the mounting member by insert molding.
